# EUROPEAN PATENT SPECIFICATION

(11) **EP 4 154 923 B1**
(45) Date of publication and mention of the grant of the patent: **17.07.2024**
(21) Application number: 22195794.7
(22) Date of filing: 15.09.2022
(51) Int. Cl.: A61L 27/44, A61L 27/54, A61L 27/56, A61L 27/58, A61L 27/60

(54) **PROACTIVE SCAFFOLD FOR THE LOADING OF ACTIVE PRINCIPLES**
PROAKTIVES GERÜST ZUM LADEN VON WIRKSTOFFEN
ÉCHAFAUDAGE PROACTIF POUR LE CHARGEMENT DE PRINCIPES ACTIFS

(30) Priority: 22.09.2021 IT 202100024296
(43) Date of publication of application: 29.03.2023
(73) Proprietor: Prolabin & Tefarm S.r.l., 06134 Perugia (IT)
(72) Inventor: SPOGLI, Roberto, 06126 PERUGIA (IT); SISANI, Michele, 06134 PERUGIA (IT); RICCIO, Michele, 60019 SENIGALLIA (AN) (IT); BONDIOLI, Elena, 47921 RIMINI (IT)
(74) Representative: Cutropia, Gianluigi

(56) References cited:
- JP-A- H1 071 199
- US-A1- 2013 211 543

## Description

### FIELD OF THE INVENTION

The present invention relates to a scaffold, of the composite type, consisting of a bio-resorbable porous matrix and of lamellar solids with ion exchange properties. Lamellar solids have the ability to absorb active ingredients in a phase prior the grafting of the scaffold and to release them after the scaffold has been grafted in an area of the patient subject to regeneration. The scaffold is suitable for use in reparative and / or reconstructive of soft tissue surgery (not bones). The scaffold can be used as dermal substitute, in wound care (surface reparative surgery), or in reparative and in depth reconstructive surgery, where a matrix able to integrate with deep soft tissues of the human body is needed.

### KNOWN ART

As it is known, some pathologies lead to the loss of portions of skin ad of subcutaneous soft tissues that can be of a certain thickness and extend up to the musculoskeletal structures. In order to resolve these inconveniences, a reconstructive surgery that uses dermic substitutes, commonly called scaffold, is known.

Pathologies related to the loss of tissues are divided in acute and chronic; the acute ones are represented by thermal, caustic and electric burns and by traumatic and post traumatic pathologies with wide and deep ulcerations. Chronic pathologies are mainly represented by degenerative lesions resulting from metabolic pathologies such as diabetes mellitus, vascular (chronic obliterating arteriopathies) and autoimmune diseases.

The prevailing therapeutic solution until the early 2000s was the use of skin grafts and / or tissue flaps, both local and transferred by microsurgical transplant.

Skin grafts produce severe scarring with aesthetic and functional consequences, both in the donor area where the tissue is taken and in the recipient area where the tissue is grafted. The skin and / or other tissue flaps also leave scarring in the donor area of the flap and are not always free from aesthetic and functional deficits in the recipient area. However, the major problem associated with the use of these traditional techniques concerns the pharmaceutical economy, as hospitalization and convalescence times are objectively long.

The introduction on the market of dermal substitutes was proposed for the solution of the above problems: aesthetic deficits, functional deficits and long hospitalization times.

The use of dermal substitutes requires indeed a very short hospitalization and does not cause serious scar damage since it does not require donor areas.

In addition, the dermal substitutes have demonstrated the ability to integrate perfectly into the host tissue, in fact they consist of collagen-based scaffolds, devoid of cellular components and therefore devoid of immunostimulating action. Dermal substitutes are rapidly vascularized by the host's neo-angiogenesis, with consequent engraftment of the graft and colonization of the dermal substitute by the host's cells. This remodelling process, which lasts about 60 days, leads to a perfect integration of the scaffold into the host's receiving tissue.

Since the advantages of using dermal substitutes are evident, in the last 20 years the surgical cases involving the use of this medical device have grown exponentially, both in the treatment of acute and chronic diseases.

However, with the growing number of cases and the consequent statistical analyzes, two critical issues related to the surgical techniques used with this technology have emerged: long integration times and infections. Infections are very serious complications in the use of these medical devices, which lead to the failure of the procedure and the loss of the dermal substitute that must be removed when infected.

In the treatment of burns and chronic metabolic, vascular and autoimmune ulcers, due to the conditions inherent in the underlying pathology, infections are frequent and harmful with consequent loss of the dermal substitute. So in these cases there is no advantage in using the device as an alternative to traditional surgical techniques (grafts and / or flaps).

In fact, the biological damage caused by the failure of the dermal substitute is associated with the economic damage, equal to the value of the dermal substitute used, and the costs of prolonged hospitalization and repeated use of operating rooms.

The dermal substitutes currently on the market guarantee integration in a minimum time of four weeks, in the absence of complications, nevertheless there is an objective need, often manifested by patients, to accelerate this integration process.

In the known art there are some exemples of bio-scaffold designed to release active ingredients after the. However, these scaffolds don't provide for the association of a bio-resorbable porous polymer and proactive lamellar solids for loading the active ingredients.

L. Moroni's pubblication (Bioactive Materials, 2021, 6(4), 1073-1082) describes lamellar solids of the hydrotalcite and zirconium phosphate chemical class preloaded with antibiotics and incorporated in a resorbable polymer via extrusion process. The composite material obtained is subsequently used for the fabrication through plasma-assisted 3D printing of scaffolds for bone regeneration capable of releasing antibiotics from lamellar solids after grafting. This scaffold is made using active ingredients (antibiotics) already loaded in the lamellar solids, so in this case the drugs are an integral part of the product and therefore are subject to the regulatory and validation process of it. JPH1071199A and JP2014030663A disclose scaffolds as dermal substitutes able to release in a prolonged way antimicrobial agents or growth factors after the grafting. Specifically, these documents describe a method for incorporating into collagen a gelatin which, following treatment with acids or bases, can assume a positive or negative charge based on the pH and its isoelectric point. This scaffold, incorporating the gelatin, can be proactive for the loading of active ingredients in a pre-operating step. However, it cannot simultaneously incorporate a positively charged gelatin and a negatively charged gelatin, therefore it cannot simultaneously load active ingredients of anionic and cationic nature. Furthermore, gelatine presents considerable problems in its approval process from the regulatory point of view, which is not the case with lamellar solids which, if used at low dosages, can be considered as formulation excipients.

In the case of JPH1071199A, in which the silver sulfadiazine is used as an active ingredient, the scaffold also has problems bound to an inhomogeneous distribution of the antibiotic in the scaffold biopolymer.

US2013211543A1 describes a hybrid three-dimensional scaffold able to support the cellular growth and the release of active pharmaceutical ingredients. However, this scaffold is exclusively suitable for bone regeneration and cannot be used as dermic substitute because it doesn't have adequate composition, mechanical properties and geometrical shape. The scaffold is in fact formed by a first biocompatible material (PLC-polycaprolacton) having the form of a structure composed of one or more interconnected voids, and by a second biocompatible material which includes a material capable of ionic exchange. The material capable of ion exchange is a phylloxylicate, or hydrotalcite (LDH) loaded with pharmaceutical ingredients. The second biocompatible material is in the form of a porous chitosan foam to anchor the lamellar solids to the porous PCL scaffold. The lamellar solids are adhered to the scaffold using an adhesive system (a binder consisting of chitosan) because the polymeric nature of the PCL and that of the lamellar solids are incompatible and it is not possible to attach them to the polymeric surface without an adhesive. The scaffold is made by fused deposition modeling with a BioScaffolder that is an additive manufacturing system using 3D-printing. The scaffold produced is a cylinder with a diameter of 10 mm, perforated, with a biopsy punch, from a 5 mm thick porous PCL mat. This scaffold has a compression stress value close to that of the target tissue (bone), therefore values of approximately 5100 kPa (capable of withstanding pressures of approximately 50 atmospheres). Subsequently, a layer of chitosan / lamellar solid intercalated with drug is deposited on this porous base.

In scientific literature there are many cases of scaffolds of bio-resorbable polymers containing lamellar solids loaded through ion exchange reactions with drugs, but there are no cases of scaffolds for tissue regeneration not loaded with drugs. In fact, the loading phase of the drug in the lamellar solids is a complex phase in which there is an ion exchange reaction between ions contained in the lamellar solid and the drug in ionic form and this phase is traditionally performed in a separate step prior to the incorporation of the drug-intercalated lamellar solids into the scaffold. Therefore, no one had thought of creating a proactive scaffold for loading drugs.

Scaffolds are known for use in restorative and soft tissues (non-bone) reconstructive surgery. However, such scaffolds are not suitable for drug loading and do not allow for slow drug release. Therefore, these known scaffolds, after implantation, have drawbacks, such as infections, capsular contracture, seroma, loss of the implant and skin necrosis. In particular, in breast surgery, the prosthetic infection rate ranges from 0.1 to 2.5% in aesthetic breast augmentation and from 1% to 35% in breast reconstruction.

The problem that arises from infections and chronic inflammation, such as to jeopardize the result of reparative and reconstructive surgery, is also found with considerable frequency in abdominal surgery, for the repair of abdominal wall lesions, in urological and gynaecological surgery for the reconstruction of the routes of excretion and of the wall of the genital organs, in neurosurgery where there is loss of substance of the dura mater or of the membranes that surround the spinal cord and the cerebral organ, in surgery of the peripheral nerves of the limbs, where the continuity of the nerve trunk must be reconstructed using the "biological tubule" technique, or in orthopaedics for the repair / reconstruction of joint surfaces injured by degenerative processes, as well as in maxillofacial surgery and dental care for the treatment of deep lesions of the mucosa capable of exposing the bone surface or for the repair of dental tissues and periodontium, and finally in cardio-vascular surgery, where the surgeon often needs to use a biological patch for the treatment of serious lesions of the heart and large arterial vessels.

### PURPOSE OF THE INVENTION

The purpose of the present invention is to eliminate the drawbacks of the prior art, providing a scaffold, suitable for use in reparative and / or reconstructive surgery of soft tissues, which is capable of loading anionic and cationic active ingredients and ensuring their slow release after plant.

Another purpose of the present invention is to provide a reliable scaffold capable of minimizing the risk of infections.

Another purpose of the present invention is to provide an efficient scaffold, capable of ensuring rapid cellular regrowth and therefore minimizing integration times into the patient's soft tissue.

These purposes are achieved in accordance with the invention with the characteristics of the attached independent claims.

Advantageous embodiments of the invention appear from the dependent claims.

### SUMMARY OF THE INVENTION

The present invention relates to a composite scaffold comprising a bio-resorbable matrix and at least one lamellar solid with ion exchange properties as defined in claim 1. Lamellar solids have the ability to selectively adsorb active ingredients in a phase prior to grafting and gradually release the aforementioned active ingredients once the scaffold has been grafted into the area subject to regeneration.

The scaffold comprises a bio-resorbable matrix comprising collagen and / or microporous hyaluronic acid. The scaffold has a leaflet shape. In this way the scaffold is soft and fluffy, so that it can be cut with hands or scissors and is suitable for implantation in a dermal lesion or in a soft tissue of a patient. The scaffold according to the invention does not require the use of a binder, because the lamellar solids adhere by chemical affinity to the surface of the resorbable polymer (collagen or mixtures of collagen and hyaluronic acid).

Natural polymers, such as collagen or hyaluronic acid, in particular have net or partial surface ionic charges and these interact with the surface potential of the lamellar solids, generating electrostatic forces of attraction for which a binder is not necessary. In some cases, the interaction between the surface of the lamellar solids and the biopolymers is so intense that ionic bonds are generated between the materials.

The composite scaffold includes:
- a bio-resorbable porous matrix, preferably variously cross-linked porous matrices, made with bovine collagen and / or porcine collagen and / or equine collagen and / or sheep collagen and / or microporous hyaluronic acid; and
- at least one lamellar solid, or mixtures of lamellar solids with ion exchange capacity, preferentially hydrotalcites (double layered hydroxides or LDH), zirconium hydrogen phosphate of alpha, gamma or lambda type, double salts of hydroxide (hydroxy double salts or HDS), phyllosilicates such as montmorillonite and hectorite.

In an embodiment of the present invention, the composite scaffold is made starting from a porous bio-resorbable matrix in the form of a pre-constituted sheet, in which the lamellar solids are incorporated by means of deposition techniques by spray-coating or by imbibition (dip-coating).

In another embodiment of the present invention, the sheet-shaped composite scaffold is made starting from a solution of bio-resorbable polymers, a suspension of lamellar solids and optionally an organic phase containing or not an emulsifier. This mixture is suitably mixed and possibly emulsified, then the solvents removed using the freeze-drying technique to obtain a microporous sheet-shaped scaffold. This scaffold can optionally be cross-linked.

The composite scaffold can be used in regenerative surgery of the dermis or injured tissues, through a procedure in which the scaffold is loaded with active ingredients in the operating room, just before being implanted in the patient.

This procedure requires that the composite scaffold is immersed and kept under gentle agitation (by shaker) or simply soaked in a solution containing the active ingredients for 10-60 minutes before being grafted. In this period of time, the active ingredients in solution are loaded into the interlayer region of the lamellar solids through ion exchange reactions or through adhesion to the surface of the lamellae with ionic or electrostatic interactions. Since the composite scaffold can simultaneously contain different classes of lamellar solids, it is proactive for loading a wide range of active ingredients of anionic or cationic nature and with molecular weight ranging from a few tens to hundreds of thousands of Dalton.

The active ingredients with which the composite scaffold can be loaded can be selected from antibiotics, anti-inflammatories, growth factors, prepared from natural substrates, antibodies, vaccines, peptides, proteins, fragments of coding nucleotides, antifungals and antimicrobials.

The advantages deriving from the slow release of the active ingredients from the composite scaffold during regeneration of the dermis or soft tissues are: the reduction of the incidence of infection, the improvement of the quality of engraftment and integration in the implant site, the ability to induce skin and soft tissue regeneration and the possibility of creating personalized therapeutic approaches by selecting suitable active ingredients for prolonged release. Compared to Moroni's publication and US2013211543A1, the present invention is distinguished in that the scaffold contains lamellar solids that are proactive to the loading of active ingredients (drugs), but not already preloaded with such active ingredients; the drugs therefore do not become part of the production and regulatory process of the scaffold according to the invention, with considerable simplification in manufacturing and marketing. In particular, the composite scaffold according to the invention is proactive for the loading of active ingredients but does not contain them and the phase of loading with active ingredients can be carried out in the operating room by the medical staff immediately before implantation of the scaffold. Moreover, compared to Moroni's paper and US2013211543A1, the present invention deals with micro porous scaffolds for soft tissues regeneration, not with bio-scaffolds for bone regeneration, areas that are very distant from the point of view of the architecture of the materials composing the scaffold and the application of the scaffold.

The scaffold of the present inventions differs from the one of US2013211543A1 as it contains lamellar solids not loaded with drugs. In addition, the lamellar solids anchor to the microporous scaffold without the need for a binder.

Compared to JPH1071199A and JP2014030663A, the present invention is distinguished by the following advantages:
- being able to incorporate both cationic and anionic active ingredients at the same time, as the lamellar solids with cationic and anionic exchange capacity can simultaneously coexist in the scaffold;
- being able to incorporate high quantities of active ingredients even with low dosages of lamellar solids;
- being able to accelerate or slow down the up-take and release kinetics of the active ingredients, modifying the type and quantity of lamellar solids in the composite scaffold;
- it is possible to obtain a uniform distribution of the lamellar solids in the porous resorbable polymer and therefore of the active principles loaded in the lamellar solids; which avoids the problems related to the non-homogeneous distribution of the active ingredients that can arise when these are incorporated directly into the polymer of the dermal substitute, as found in JPH1071199A in the case of silver sulfadiazine;
- manufacturing advantages, as the incorporation of lamellar solids into the dermal substitute can be easily achieved both in the scaffold construction step (via freeze-drying), and by applying a suspension of lamellar solids in a pre-established scaffold through imbibition techniques or spray-coating.

With respect to the state of the art, it is remarkable that the composite scaffold according to the invention is suitable for loading not only an active ingredient or a few active ingredients, but also for loading complex compositions containing multiple active ingredients, such as those obtained from colostrum and placenta extracts or extracts from plant products. Such complex compositions can be loaded in a single step into the dermal substitute and released slowly over time during the regeneration period, resulting in a significant improvement in the quality and speed of the regenerative process.

### BRIEF DESCRIPTION OF THE FIGURES

Fig. 1A is a drawing of the composite scaffold according to the invention consisting of a porous and bio-resorbable matrix and lamellar solids in the form of light grey platelets;
Fig. 1B is a schematic representation of the structure of a generic lamellar solid with exchangeable ions (in the shape of balls) in the interlayer region;
Fig. 1C is a schematic representation illustrating the loading of a solution containing active ingredients into the composite scaffold of Fig. 1A by imbibition;
Fig. 1D is a schematic representation illustrating the grafting of the composite scaffold of Fig. 1A into a patient's wound and the activation of the release of active ingredients from the composite scaffold to the patient's dermis;
Figs. 2A and 2B are two SEM scanning electron microscope images, showing an enlargement of the outer surface and cross section of a sheet of a single-layer Pelnac-type commercial dermal substitute, respectively;
Figs. 3A and 3B are two SEM scanning electron microscope images, showing respectively an enlargement of the external surface and of the cross section of a dermal substitute according to the invention consisting of a bio-resorbable porous matrix loaded with a mixture of lamellar solids of Example 2;
Fig. 4 is an enlarged detail of Fig. 3B;
Fig. 5 shows two SEM images of a magnesium-aluminum hydrotalcite containing chloride anions in the interlayer region (material produced by Prolabin & Tefarm code HT-102; AEC = 3.7 meq/g); and
Fig. 6 shows images of in vitro biological tests (example 6) on the bacterial decontamination capacity of a scaffold according to the invention and a scaffold according to the known technique.

### DETAILED DESCRIPTION OF THE INVENTION

The scaffold according to the invention comprises a bio-resorbable porous matrix and at least one lamellar solid with ion exchange capacity.

The porous bio-resorbable matrix has a resorbing speed in the patient's skin site equal to a maximum of 28-30 days.

The porous bio-resorbable matrix has a texture that can be simple porous, windowed with regular windows or with windows of variable size.

The porous bio-resorbable matrix has a porosity with pores having an average diameter from 1 µm to 1000 µm or more preferably between 50 µm to 200 µm.

The bio-resorbable porous matrix comprises a biopolymer or a mixture of biopolymers including, by way of example but not limited to, collagen of bovine origin, collagen of porcine origin, collagen of equine origin, collagen of sheep origin, hyaluronic acid. Hyaluronic acid can be of low, medium or high molecular weight. Each biopolymer can be cross-linked in various ways.

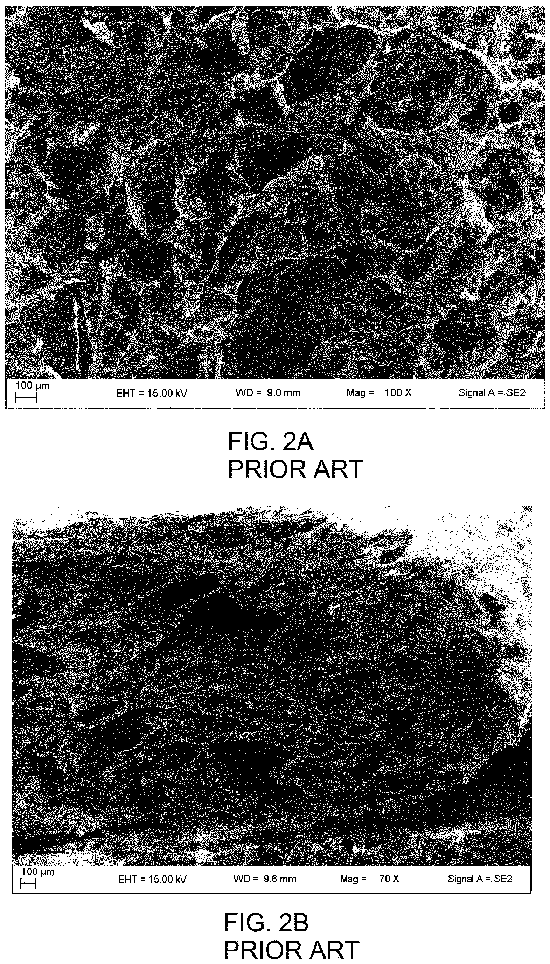

Fig. 2 shows SEM microscope images of a bio-resorbable porous matrix, in this specific case the dermal substitute commonly available on the market under the brand name PELNAC^{®}. In these images the pores of the matrix are clearly visible.

In the context of the present invention, by lamellar solid is meant an inorganic compound not containing organic carbon, which, due to its chemical structure, forms a solid that is spatially arranged as a succession of lamellae, i.e. planar macrocrystals having a size much lower (thickness) than the other two (see Chapter 1 of Volume VII of Comprehensive Supramolecular Chemistry, Pergamon Press, Oxford, 1996).

In the context of the present invention, a lamellar solid with ion exchange capacity means a lamellar solid in which the lamellae have an electric charge (positive or negative) inside the tunnels, or between the lamellae, counterbalanced by ions of opposite charge to preserve the electroneutrality of the solid. This electric charge can be localized on the lamella, in the case of hydrotalcites and double hydroxide salts, or localized on functional groups anchored to the lamella (as in the case of zirconium hydrogen phosphate). The ions that counterbalance the charge of the lamellae are located in the interlamellar region and can be replaced through ion exchange reactions by other ions with a speed and selectivity linked to factors such as: ionic concentration, steric effect of the ions, net charge of the ion, specific chemical affinity of the ion towards the lamellar matrix. The composite scaffold according to the invention contains lamellar solids in which the ions located in the tunnels are easily exchangeable through ion exchange reactions, that is to say are suitable or pro-active for the loading of other ions including the anionic and cationic active ingredients.

In lamellar solids with ion exchange capacity, this property is quantified through the Ionic Exchange Capacity (IEC), this quantity is directly proportional to the capacity of loading of active ingredients. The IEC is a quantity defined by the amount of exchangeable ions that a material can retain by ion exchange. In the case of lamellar solids with positive charges localized on the lamella we speak specifically of anionic exchange capacity (AEC). AEC is expressed in milliequivalents per gram (meq / g) or in milliequivalents per 100 g (meq / 100 g) of material (see Surface and Interface Chemistry of Clay Minerals, Volume 9, 2018, Editors: Robert Schoonheydt Cliff Johnston Faïza Bergaya in Developments in Clay Science). In the case of lamellar solids with negative charges localized on the lamella we speak specifically of cationic exchange capacity (CEC). The similar to AEC, is expressed in milliequivalents per gram (meq / g) or in milliequivalents per 100 g (meq/100 g) of material.

In the context of the present invention, the lamellar solids with ion exchange capacity preferentially belong to the following chemical classes: hydrotalcites (LDH), hydroxide double salts (HDS), zirconium hydrogen phosphate of the alpha, gamma or lambda type and phyllosilicates. These types of lamellar solids have been selected as they have a high IEC, where high IEC means a value greater than 1 meq/g. In particular, typical IEC values for the selected are the following: zirconium hydrogen phosphate of the alpha, gamma or lambda type CEC=6.3-6.6 meq/g; LDH AEC=2.7-4.2 meq/g, HDS AEC= 3.0-4.5 meq/g; phyllosilicates (montmorillonite) CEC=1.0-1.5 meq/g; phyllosilicates (hectorite) CEC= 1.4-1.6 meq/g.

The higher the IEC of the lamellar solid, the greater the theoretical loading of active ingredients in the solid and consequently in the composite scaffold.

It has to be noted that the selected lamellar solids, compared to other solids with ionic exchange capacity such as the zeolites (CEC = 2-4 meg/g), disperse very well in aqueous suspensions, therefore, when they are used in the production of composite materials they do not run into problems related to the non-uniform distribution of the particles, difficulties that are commonly encountered with non-lamellar inorganic materials. Furthermore, another limitation of zeolites is that they are not able to exchange ions larger than the available cavity (determined by the typical cage structure of zeolites), while the lamellar solids can enormously increase the interlayer distance and insert ions of large dimensions without steric bulk problems. Thus lamellar solids allow for a much wider variety of molecules and active ingredients to be loaded than zeolites.

The lamellar solids according to the invention are characterized by the following formulas:
1) Hydrotalcites (double layered hydroxides or LDH)

   General formula [M(II)₁₋ₓM(III)ₓ(OH)₂]^{x+}(Aⁿ⁻)_{x/n}^{*} mH₂O

   where
   M(II) is a bivalent metal, i.e.: Mg, Zn, Fe, Co, Ni, Mn, Cu, Ca;
   (III) is a trivalent metal, i.e.: Al, Fe, Co, V;
   the molar fraction of the cation x is expressed as the ratio M(III)/[(MII) + (MIII)] (generally 0,2 ≤ x ≤ 0,4);
   A can be an organic or inorganic anion with a charge n- (where n = 1, 2, 3, 4);
   m is the number of water moles for formula unit (generally 0,4 ≤ m ≤ 1).
   Within the scope of the present invention, hydrotalcites with M(II)= Mg, Zn; M(III)=Al are particularly relevant for the biocompatibility of materials; those with A= NO₃⁻, Cl⁻, CH₃COO⁻, I⁻, Br, CO₃⁻² are relevant for obtaining proactive lamellar solids for loading with active ingredients
2) hydroxide double salts (HDS)

   General formula: [M(II)₁₋ₓM(II)₁₊ₓ(OH)_{3(1-y)}]⁺(Aⁿ⁻)_{(1+3y)/n} * mH₂O

   where
   M(II) can be Mg, Zn, Fe, Co, Ni, Mn, Cu, Ca; (with 0 ≤ x < 1; 0 ≤ y<1);
   A can be an organic or inorganic anion with a charge n- (where n = 1, 2, 3, 4);
   m is the number of water moles for formula unit (generally 0,5 ≤ m ≤ 1).
   Within the scope of the present invention hydroxide double salts with M(II)= Mg, Zn, Fe, Ca are particularly relevant for the biocompatibility of materials; those with A = NO₃⁻, Cl⁻, CH₃COO⁻, I⁻, Br⁻, CO₃⁻² are relevant for obtaining proactive lamellar solids for loading with active ingredients
3) Phyllosilicates

   General formulas: A₃Si₂O₅(OH)₄ e A₃Si₄O₁₀(OH)₂

   where A can be Ca, Mg, Al, Na, Fe, Ti, Li, K, Ba;
   Within the scope of the present invention, the phyllosilicates belonging to the montmorillonite, hectorite and micas family are particularly relevant for biocompatibility, ease of dispersion in water and for obtaining proactive lamellar solids for loading with active ingredients.
4) zirconium hydrogen phosphate of the alpha, gamma or lambda type. Phosphate general formula Zr(IV)(RPO₄)₂

Within the scope of the present invention those with R = H⁺, K⁺, Na⁺, Li⁺, Ca²⁺, NH₄⁺, or an organic alkyl or aryl group are particularly relevant for biocompatibility and for obtaining proactive lamellar solids for loading with active ingredients.

The lamellar solids according to the invention are powders with particle sizes in terms of D₉₀ between 350 nm and 20 µm.

The lamellar solids according to the invention are characterised by an AEC or a CEC between 1.0-10 meq/g.

The lamellar solids according to the invention are characterised by a surface area (BET) between 1-200 m²/g.

The percentage by weight of lamellar solids present in the composite scaffold is comprised between 0.8-4.0%.

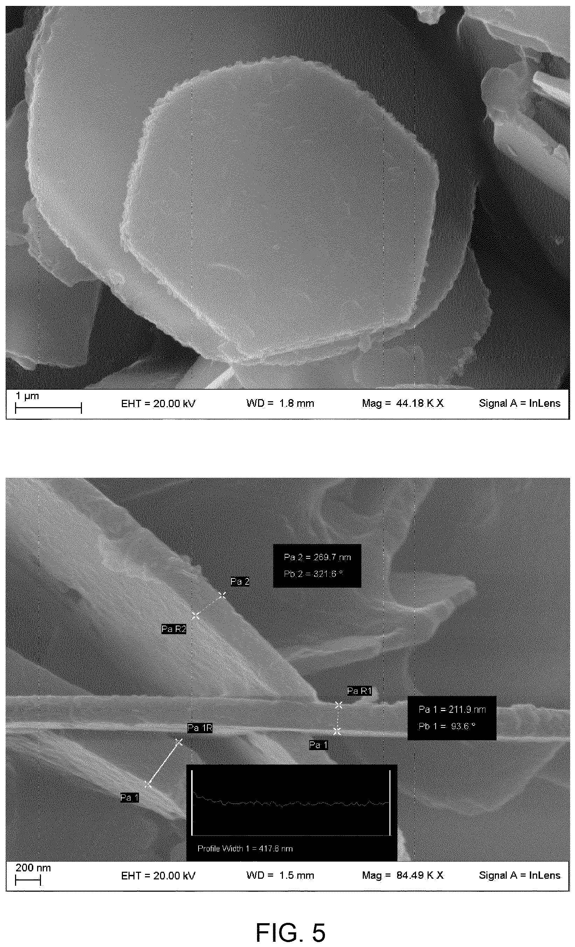

Fig. 5 shows by way of example the SEM microscope images of a lamellar solid used in the invention. It is a magnesium-aluminum hydrotalcite balanced in the interlayer region by chloride anions (material produced by Prolabin & Tefarm code HT-102; AEC = 3.7 meq / g).

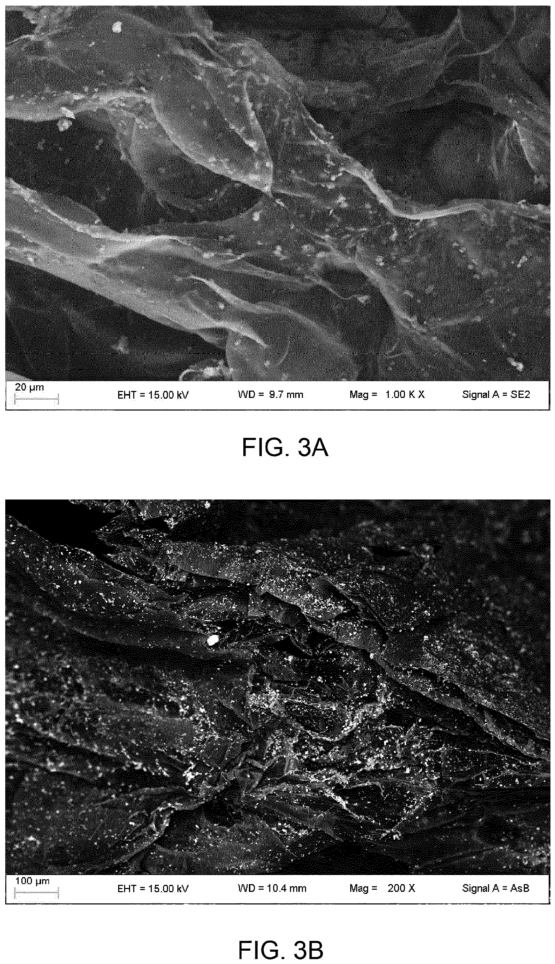

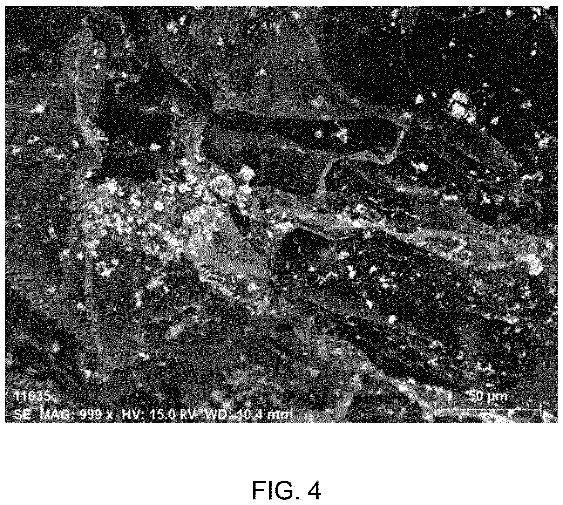

Figs. 3A, 3B and 4 show the SEM microscope images of the bio-resorbable porous matrix (illustrated in Fig. 2) loaded with 15% by weight of lamellar solids comprising 70% of Layered Double Hydroxides LDH and 30% of zirconium phosphate (ZrP) and obtained by means of the imbibition process (product obtained in example 1). As can be seen in Figs. 3A, 3B and 4, the lamellar solids (which have the shape of white dots / hexagons) are well distributed in the porous matrix, precisely because they penetrate inside the pores.

Advantageously, the bio-resorbable porous matrix can be loaded with a mixture of lamellar solids characterized by both high cation exchange capacity (CEC) and high anion exchange capacity (AEC).

Some methods for obtaining the composite scaffold according to the invention are described below.

The composite scaffold according to the invention has the shape of a sheet having a thickness of 0.1 - 20 mm.

In an embodiment of the present invention, the composite scaffold is produced starting from a pre-constituted bio-resorbable porous matrix in the form of a sheet in which the lamellar solids are incorporated by means of deposition techniques by spray-coating or by imbibition.

The preconstituted bio-resorbable porous matrix can be, by way of example, the single layer Pelnac type with a thickness of 2.5 mm, marketed by Gunze Limited, which is made up of atelocollagen derived from ultrapure type I porcine tendon with a porosity of 70- 110 µm.

The aforementioned processes initially provide for the preparation of a suspension of at least one lamellar solid in a solvent. The preferred solvent for making the suspension is water, however mixtures of water and water-miscible organic solvents such as alcohols, tetrahydrofuran, acetonitrile, acetone, dioxane, dimethyl sulfoxide or other organic solvents such as ethyl acetate, ethers or aromatic compounds can also be used.

The total concentration of the lamellar solid or solids in the suspension is between 100-0.1 mg / ml or more preferably between 10-1.0 mg / ml. The lamellar solids are homogeneously dispersed in the solvent by subjecting the suspension to treatments such as: turbo-mixing and / or ultrasound and / or high pressure homogenization. To properly prepare the dispersion of the lamellar solids at the application concentration, a concentrated suspension is started which is subjected to alternating cycles of dispersing and dilution treatments (see example 1). Once the suspension has been prepared, it is kept under gentle agitation (40-100 rpm) until the next step.

In the imbibition process, the suspension of the lamellar solids is applied drop by drop on the pre-made matrix through a syringe or similar instrument and using a volume such that all the suspension is absorbed by the pre-made matrix. In the case of single-layer S-type Pelnac 2.5 mm thick, a volume of approximately 3 ml of suspension per 100 mg of matrix is used (approximately 0.4 ml/cm²). Using this procedure, it is possible to easily load the matrix up to 150% of its weight with lamellar solids.

In the spray-coating process, the suspension of the lamellar solids is applied by means of a spraying technology on one of the sides of the pre-made matrix. The spraying technologies can be those that use an airbrush, an automatic spray coater or similar. The concentration of the lamellar solids in the sprayed suspension can reach 5% by weight of lamellar solids (about 50 mg / ml), but more preferably a concentration of 0.5% by weight of lamellar solids is used (about 5 mg / ml). Using this procedure, it is possible to easily load the matrix up to 20% of its weight with lamellar solids.

The pre-made matrices loaded with the suspension of lamellar solids through the two procedures described above are subsequently subjected to a solvent removal process.

To obtain sheets with a clear shape and smooth surface, the matrices can be housed in suitable shaped containers (plastic or steel trays) for the solvent removal process. The solvent can be removed through freeze-drying techniques, evaporation at reduced pressure in the presence of desiccants, evaporation in an oven.

Once the solvent is removed, the lamellar solids adhere to the protein surface of the pre-made matrix through ionic and electrostatic interactions. These interactions keep the lamellar solids firmly bonded to the surface of the matrix both on the outside and in the internal cavities of the microporous structure (see Figs. 3A, 3B and 4). From the SEM photos it is possible to see that the lamellar solids are uniformly distributed in the section of the matrix.

To increase the adhesion of lamellar solids to the matrix and control the level of residual moisture, it is possible to isothermally treat the matrix in a ventilated oven at 105 °C for 2-24 hours.

The obtainment of the composite scaffold from pre-made matrices can also be achieved using pre-made double-layer matrices in which there is, in addition to the bio-resorbable porous polymer layer, also another layer, generally silicone, with the aim of promoting the drainage of the wound and give greater strength to the scaffold. In the case of pre-made double-layer matrices, the loading process of the lamellar solids by imbibition or by spray-coating takes place on the side consisting of the bio-resorbable porous polymer.

In another scope of realization of the present invention, the sheet-shaped composite scaffold is made integrally starting from the primary constituents in a process as detailed below:
1. preparation of a phase consisting of one or more bio-resorbable polymers solubilized in a solvent;
2. preparation of a phase consisting of a suspension of
3. one or more lamellar solids in a solvent;
4. preparation of a phase consisting of an organic solvent such as chloroform;
5. emulsion and homogenization or mixing of the previous phases;
6. drying by freeze-drying;
7. thermal or chemical crosslinking.

The preferred solvent for the preparation of steps 1 and 2 previously described is water, but mixtures of water and water-soluble solvents such as alcohols, acetone, tetrahydrofuran, acetonitrile, dioxane and dimethyl sulfoxide can also be used.

In preparations in which the biopolymers are solubilized in water, after the solubilization of the biopolymers the pH is adjusted to a suitable value such as for example in the range between 2.5 and 5.5.

Steps 4 and 7 of the previously described preparation can be optional.

From a regulatory point of view, in the dermal substitute according to the invention and according to the specific legislation of the country, the lamellar solids can be advantageously considered as excipients of the formulation based on collagen or other bio-polymer, simplifying the process of approval of the biomedical product.

This assertion is supported by the following data:
- low content of lamellar solids in the scaffold;
- chemical inertness of lamellar solids;
- lamellar solids do not bio-accumulate in the tissues.

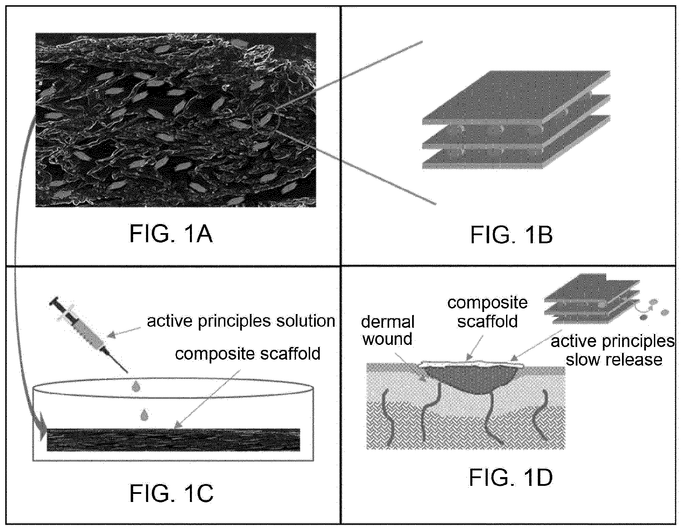

Fig. 1B shows the general structure of a lamellar solid, consisting of positively or negatively charged layers and ions of opposite charge in the interlayer region that maintain the electroneutrality of the solid. In Fig. 1B the ions are represented with balls. Each particle of lamellar solid is made up of hundreds of layers that form a "pillared" structure (see Fig. 5). In addition to the anions there are solvent molecules, generally water, in the interlayer region.

The lamellar solids according to the invention are preferably those belonging to the class of hydrotalcites (layered double hydroxides), double hydroxide salts, zirconium phosphate of the alpha, gamma and lambda type, hectorites and phyllosilicates.

Furthermore, the lamellar solids according to the invention are characterized by an IEC> 1 meq / g and by ions in the interlayer region that are easily exchangeable through ion exchange reactions, including but not limited to: NO₃⁻, Cl⁻, CH₃COO⁻, I⁻, Br-, CO₃⁻², H+, K+, Na⁺, Li+, Ca²⁺, NH⁴+, OH⁻. Since the lamellar solids are characterized by easily exchangeable ions in the interlayer region, they can be considered proactive for the loading of other ions which in this context are constituted by the active principles.

Fig. 1A is a cross-sectional representation of a dermal substitute according to the invention consisting of a resorbable porous matrix, such as type I collagen and lamellar solids incorporated into the matrix. The incorporation of the lamellar solids can be obtained with one of the manufacturing processes described and the lamellar solids are either adhered to the surface of the bio-resorbable polymer (processes by imbibition or spray-coating) or incorporated into its structure (process for freeze-drying).

Fig. 1C shows the procedure for loading active ingredients into the composite scaffold for imbibition. This procedure foresees that the composite scaffold is put in contact with a solution of active ingredients of an ionic nature. Such active principles of an ionic nature can be salts, zwitterions or molecules with an acid or basic character or any kind of molecule having a net positive or negative charge. The loading process of the active ingredients in the composite scaffold proceeds through ion exchange reactions between the ions of the interlayer region and the active ingredients of an ionic nature.

The procedure described in Fig. 1C can be carried out in a pre-operative step by immersing the composite scaffold in a solution containing the active ingredients or by depositing the solution on the composite scaffold with a syringe (imbibition). This step can be advantageously carried out in the operating room by the medical staff by immersing the composite scaffold in a dish containing a solution of active ingredients or by soaking the composite scaffold with a syringe containing the solution of active ingredients. The duration of contact between the solution of the active ingredients and the composite scaffold can be 10-60 minutes. Furthermore, the composite scaffold soaked in the solution of active ingredients can be grafted into the wound immediately after the soaking process, since the ion exchange reactions that load the active ingredients into the lamellar solids of the scaffold proceed in the first minutes following grafting, as long as the solution of active ingredients does not spread into the surrounding tissues.

The composite scaffold can be loaded with mixtures of lamellar solids of different types to favor the rapid and effective loading of chemically different anions and cations and with distinct and synergistic biological functions.

Fig. 1D shows the situation where the composite scaffold after loading with the active ingredients is grafted into a patient wound. In the situation described, the composite scaffold gradually comes into contact with extracellular fluids and wound exudate, which contain a significant anionic and cationic charge. The ions of the extracellular liquids and of the exudate, crossing the scaffold in the cellularization process, meet the lamellar solids loaded with the active ingredients and, due to the effect of ionic concentration and affinity towards the matrices, activate the ion exchange reactions that lead to the slow release of the active ingredients from the composite scaffold.

This ion exchange is slow-release and can last for more than ten days.

The active ingredients can be substances suitable for avoiding infections after grafting the composite scaffold. In this case, the active ingredients may comprise one or more of the following active ingredients: antibiotics, antifungals, antimicrobial agents including antiseptic and disinfectant agents, antibodies.

Among the antibiotic agents, all those of anionic or cationic type can be used including, but not limited to, gentamicin, streptomycin, benzylpenicillin, phenoxymethylpenicillin, ampicillin, ciprofloxacin, nalidixic acid, amphotericin B, chloramphenicol, cefazolin, amoxicillin, norfloxacin, benzoate, succinate and ticarcillin.

Among the antifungals, itraconazole and amphotericin B can be used by way of example.

Among the antiseptic agents, potassium permanganate, boric acid, anionic phenols, hypochlorite, copper sulfate, hypochlorites, chlorhexidine, merbromin, iodides, bromides, salicylic acid, octaphonium and other quaternary ammonium salts can be used.

Among the disinfectant agents, quaternary ammonium salts, guanidines, halogens, aldehydes and detergents can be used.

Engineered antibodies can also be used among antibodies.

The active ingredients can be substances suitable for strengthening the patient's immune defenses against viruses and bacteria. In this case the active ingredients include antibodies, such as immunoglobulins and vaccines.

The active ingredients can be substances suitable for assisting the skin regeneration of soft tissues in the graft area of the composite scaffold. In this case the active ingredients may include anti-inflammatories, growth factors, prepared from natural substrates.

Among the anti-inflammatories diclofenac, ibuprofen, naproxen, ketoprofen, fenbufen, flurbiprofen, indomethacin, mefenamic acid, meclofenamic acid and dexamethasone or other steroid derivatives can be used.

Growth factors include basic fibroblast growth factor (bFGF), insulin-like growth factor 1 (IGF-1) and platelet-derived growth factor (PDGF) as examples.

Among the preparations from natural substrates, those from the placenta or colostrum can be used.

The active ingredients may include peptides and/or proteins such as glycoproteins, lipoproteins, cytokines, fragments of coding nucleotides including DNA, RNA and plasmids. These active ingredients accelerate the cell regeneration process and improve the quality of the tissue.

It must be considered that the composite scaffold according to the invention is capable of simultaneously loading several active ingredients of anionic or cationic type to obtain a combined therapeutic effect: the hypothetical example of a scaffold loaded with growth factors and other active ingredients such as antibiotics and anti-inflammatories is reported.

Preferably, the composite scaffold is made first, comprising the porous matrix and the mixture of lamellar solids; subsequently it is immersed in a solution containing the active principles in order to incorporate them in the lamellar solids and then in the composite scaffold. In this way, the active ingredients can be added immediately before grafting the composite scaffold, according to the patient's personal needs.

This entails a series of advantages with respect to the known art. In fact, there is no pre-packaged scaffold with active ingredients, but the active ingredients can be selected by the medical staff and loaded at the time of grafting.

Compared to the prior art, the composite scaffold according to the invention is characterized by a greater loading capacity of active ingredients. In case the scaffold is loaded with 5% by weight of lamellar solids, it can load and then release approximately up to 100 mg/cm² of active ingredients.

The dermal substitute may further comprise a film of synthetic material removably applied to the sheet of the composite scaffold comprising the porous matrix and the lamellar solid. The film of synthetic material can be porous or windowed and has the purpose of slowing the dehydration of the wound by offering a barrier to water vapor, increasing the mechanical strength of the composite scaffold and promoting drainage of exudate.

When the dermal substitute comprising the film of synthetic material is applied to the skin, the film remains facing outwards.

After about 3-4 weeks from grafting, since the scaffold is reabsorbed by the skin, the film can be removed.

The film can be made of elastomeric silicone or medical grade microporous silicone and can be 0.1 - 1 mm thick.

The adhesion of the film with the sheet of the composite scaffold takes place by means of an electro-chemical or thermo-induction process or through the use of biocompatible adhesives.

The composite scaffold in the case of deep lesions can also be used for the regeneration of patients' soft tissues, in this case a scaffold without the plastic film is used.

In the context of the present invention, the composite scaffold can be used not only in the human field but also in the veterinary field.

### EXAMPLES

### Materials

All materials used in the examples, unless otherwise noted, were purchased from Merck (Sigma Aldrich).

The lamellar solids used are those commercially available from Prolabin & Tefarm S.r.l. (Italy) with the codes and characteristics specified below:
Hydrotalcite (LDH) Zinc aluminum nitrate: commercial code: HT-203; AEC = 2.7 meq/g; formula: [Zn₂Al(OH)₆](NO₃) · H₂O; crystallographic pattern: trigonal crystal system; interlayer distance = 8.9 Å (planes 003); particle size distribution: D90 <20 µm, D50 <10 µm; BET specific surface area: 5-25 m²/g.

Alpha- Zirconium hydrogen phosphate: commercial code: ZP-101; CEC = 6.6 meq/g; formula: Zr(HPO₄)₂ · H₂O; crystallographic pattern: monoclinic crystalline system interlayer distance = 7.5 Å (planes 002); particle size distribution: D90 <10 µm, D50 <5 µm; BET specific surface area: 5-15 m²/g.

Zinc hydroxy nitrate (HDS): code HN-100; AEC = 3.2 meq/g; Formula: Zn₅(OH)₈ (NO₃)₂ · 2H₂O; crystallographic pattern: monoclinic crystalline system interlayer distance = 9.6 Å; particle size distribution: D90 <20 µm, D50 <10 µm; BET specific surface area: 1-20 m²/g

Hydrotalcite (LDH) Magnesium aluminum chloride: code HT-102; AEC = 3.7 meq/g; formula: [Mg₂Al(OH)₆](Cl) · H₂O; crystallographic pattern: trigonal crystal system interlayer distance = 7.8 Å (planes 003); particle size distribution: D90 <15 µm, D50 <10 µm; BET specific surface area: 5-25 m²/g.

Hydrotalcite (LDH) Magnesium aluminum carbonate; code: HT-101; AEC = 3.7 meq/g; formula: [Mg₄Al₂(OH)₁₂](CO₃) · H₂O; crystallographic pattern: trigonal crystal system, interlayer distance = 7.6 Å (planes 003); particle size distribution: D90 <10 µm, D50 <5 µm; BET specific surface area: 5-20 m²/g.

Hectorite: code: PH-300; CEC = 1.4 meq/g; formula: Hi₂Li₂Mg₁₆Na₂O₇₂Si₂₄; particle size distribution: D90 <250 µm, D50 <100 µm; BET specific surface area: 350-390 m²/g.

Activated Bentonite: code: PH-100; AEC = 1 meq / g; formula: (Na,Ca)_{0.3}(Al,Mg)₂Si₄O₁₀(OH)₂ · n(H₂O); crystallographic pattern: monoclinic crystal system, interlayer distance = 12.6 Å; particle size distribution: D90 <50 µm, D50 <25 µm; BET specific surface area: 700-800 m²/g.

Water: the water used in all the examples is type 3 according to ISO 3696:1987, obtained through a process of microfiltration, reverse osmosis and sterilization with a UV-VIS lamp.

Collagen: the collagen used for the manufacture of the composite scaffold is type-I, in micro-fibrils, derived from Achilles tendon of bovine origin: Collagen from bovine achilles tendon, Sigma Aldrich, code C9879.

Hyaluronic acid: the hyaluronic acid used for the manufacture of the composite scaffold is: Hyaluronic acid sodium salt from bovine vitreous humor, Sigma Aldrich, Code H7630.

Pre-established dermal substitute: Pelnac Type S 60 * 82 mm and 2.5 mm thick purchased from GUNZE was used, consisting of atelocollagen derived from ultrapure type I porcine tendon with porosity of 70-110 µm.

PSA (Penicillin-Streptomycin-Amphotericin B Solution): marketed by Biological Industries as a 100 ml solution containing 1 gram of streptomycin, 0.0025 gr of Amphotericin B sulfate and 1,000,000 Units of penicillin G sodium salt 10,000 units/ml Penicillin G Sodium Salt; pH solution about 6.9.

### Measuring instruments

Particle size: the particle size distribution was obtained with a Malvern Mastersizer 2000 with Scirocco and Hydro G.

SEM images: SEM images were obtained using a LEO 1525 Field Emission Scanning Electron Microscope (FE-SEM) equipped with an EDX Bruker probe.

XRPD spectroscopy: the measurements were carried out with the Bruker D2 Phaser 2nd generation X-Ray Powder Diffractometer, equipped with a LynxEye SSD160 solid state detector and using Cu Kα as a radiation source.

### Process equipment

Freeze-drying: the instrument used is the Virtis Advantage El Freeze Dryer Lyophilizer System model, from Biopharma Process System.

Ultrasonic bath: the instrument used is the VWR ULTRASONIC CLEANER model, power 80W, frequency 45KHz.

Spray-coating: the instrument used is the SonoTek automatic countertop type ExactaCoat Spray Coater system.

Homogenizer: the instrument used is the ultra turrax IKA T25 DIGITAL model.

Ultra-centrifuge: the instrument used is the ROTANTA 460 HETTICH ZENTRIFUGEN model.

EXAMPLE N ° 1: production method of the composite scaffold through the soaking technique.

The use of this method allows to obtain a dermal substitute sheet containing one or more lamellar solids deposited on the internal and external surface of the porous sheet. The lamellar solids are conveyed inside the dermal substitute using an appropriate aqueous suspension with which the leaflet is soaked.

The ratio of mass of dermal substitute and water used has been optimized and corresponds to the amount of water that the dermal substitute can completely absorb homogeneously. In the case of Pelnac Type S 2.5 mm thick, the ratio between Pelnac weight and suspension volume is 3.0 ml of lamellar solids dispersion per 100 mg of dermal substitute (3 ml/100 mg); this ratio can also be expressed as 0.40 ml per cm². Below is the detailed procedure:
step 1- Preparation of mother suspension N ° 1.
   72.2 mg of hydrotalcite of zinc aluminum nitrate type (code HT-203) and 30.1 mg of zirconium hydrogen phosphate code (ZP-101) are weighed and introduced into a 5 ml glass vial with screw closure, subsequently 2.0043 g of water are introduced in the vial. The obtained white dispersion is sonicated for 15 minutes in an ultrasonic bath. The concentration obtained is 51.0 mg/ml.
step 2- Preparation of stock suspension N ° 2. This step involves a 1/10 dilution of stock suspension N ° 1.
   0.2074 g of stock suspension N° 1 is weighed in a 5 ml glass vial with screw cap, then 1.8032 g of deionized water is added. The obtained dispersion is sonicated in an ultrasonic bath for 15 minutes. The concentration of total lamellar solids obtained in the aqueous dispersion is 5.00 mg/ml. The suspension is kept in gentle agitation using a shaker until use in step 3.
step 3- Imbibition of the Pelnac leaflet. A square of about one cm² and weighing 14.1 mg of a Pelnac type S sheet is placed on a Petri dish; an amount equal to 0.423 g of the type N ° 2 mother suspension is uniformly deposited on the surface of the leaflet by means of an insulin syringe. The droplets deposited, which initially settle on the surface, are absorbed in 10-30 s giving rise to a homogeneously soaked Pelnac sheet, which retains its original shape and retains all the aqueous suspension used.
step 4- Drying of the soaked Pelnac. The Pelnac sheet soaked in the previous step is dried by freeze-drying with a procedure that provides for the cooling of the sample with a ramp from 0.5 ° C/min up to -20 °C, stop for 2 hours at -20 °C and subsequent sublimation of ice at 0 °C to 10.7 Pa (80 mTorr) for 20 hours. The material obtained maintains the original shape of the Pelnac sheet. The weight of the material obtained after drying is 16.5 mg corresponding to a mass increase of about 15% due to the addition of lamellar solids.

EXAMPLE N ° 2: production method of the composite scaffold through the soaking technique - variant in drying.

This example involves the use of the same procedure used in example 1 with the exception of the drying step which is carried out at reduced pressure (533.3 Pa (4 Torr)) 22 °C for 15 hours and the presence of a desiccant such as phosphorous anhydride.

### EXAMPLE N ° 3

This example uses the same procedure as in example 1 applied to different compositions of lamellar solids as shown below:
composition 1: hydrotalcite magnesium aluminum chloride, (code HT-102) (70% by weight), zirconium hydrogen phosphate (code ZP-101) (30% by weight);
composition 2: hydrotalcite magnesium aluminum chloride, (code HT-102) (100% by weight);
composition 3: hydrotalcite magnesium aluminum carbonate (code HT-101) (50% by weight), activated bentonite, (code PH-100) (20% by weight), hectorite (code PH-300) (30% by weight).

EXAMPLE N ° 4: method of production of the composite scaffold through the spray coating technique
step 1- Preparation of the mother suspension: the same method as in example 1 is used, obtaining a dispersion of hydrotalcite zinc aluminum nitrate/alpha zirconium hydrogen phosphate in a ratio of 70/30 and with a concentration of 15.0 mg/ml. The suspension is kept in gentle agitation using a shaker until it is used in the next step.
step 2- Spray-coating on Pelnac sheet. A square sheet of 5 cm per side and weighing 360.2 mg, obtained by cutting out a unit of Pelnac type S, is placed on a Petri dish; a quantity equal to 100 mg/cm² of suspension prepared in step 1 is then deposited by spraying using an automatic ExactaCoat Spray Coater system.
step 3- Drying. The sprayed sheet is dried by freeze-drying by cooling the sample with a ramp from 0.5 °C/min down to -20 °C, pausing for 2 hours at -20 °C and subsequent sublimation of the ice at 0 °C at 10.7 Pa (80 mTorr) for 20 hours. The weight of the material obtained after drying is 397.6 mg corresponding to a mass increase of about 10% due to the addition of lamellar solids.

EXAMPLE N ° 5 - production method of the composite scaffold with the freeze-drying technique
step 1- Preparation of the resorbable polymer solution.

Using deionized water and 0.5 M hydrochloric acid, a pH 3.0 solution is prepared containing 0.4% by weight of collagen of bovine origin. Subsequently, hyaluronic acid is added and solubilized in a concentration equal to 0.1% by weight. The total concentration of collagen and hyaluronic acid is therefore equal to 0.5% by weight.

Step 2- Preparation of suspension containing lamellar solids. A 200 g aliquot of solution prepared in step 1 is subjected to a pH correction process using hydrochloric acid and 0.01 M sodium hydroxide to obtain a solution with a pH between 6.0 - 7.5. A mixture of lamellar solids weighing 0.150 g and composed of 70% by weight of zinc hydroxy nitrate (code HN-100) and 30% by weight of zirconium hydrogen phosphate (code ZP-101), is added to the 200 g of solution, homogenized for 5 minutes and then subjected to treatment in an ultrasonic bath for 15 minutes.

Step 3- Preparation of the emulsion. Chloroform is added to an aliquot of the suspension obtained in step 2 to obtain a concentration equal to 2% by weight; the mixture is then homogenized with ultra-turrax and centrifuged at 7000 rpm for 5 minutes to remove air bubbles.
step 4- Obtaining the porous scaffold for freeze-drying. An aliquot of the emulsion obtained in step 3 is poured into a flat stainless steel mold, placed on the shelf of the freeze-drying apparatus and cooled using a 0.5 °C/min ramp down to -20 °C. The temperature is maintained for two hours to complete freezing, then the ice is sublimated by treatment at 0 °C and 1 Pa (80 mTorr) for 20 hours. The freeze-drying process produces a highly porous polymer matrix with a thickness of 2-3 mm which is subjected to crosslinking by treatment at 105 °C at 1.3 Pa (10 mTorr) for 24 h.
step 5 (optional) - Lamination with silicone layer and crosslinking. The porous polymer sheet obtained in step 4 is coated with a silicone layer of about 0.3 mm thickness and then subjected to treatment in 0.05 M acetic acid at room temperature for 24 hours. The matrix is further cross-linked by a 24-hour treatment with a solution of 0.25% (v/v) glutaraldehyde in 0.05 M acetic acid. The bilayer sheet is then washed five times for 5 minutes with distilled water and subjected to freeze-drying as in step 4.

EXAMPLE N ° 6 - In vitro biological tests on bacterial decontamination capacity.

The tests were carried out using a scaffold prepared according to example 1 and a commercial single-layer Pelnac-type scaffold as a reference. The test measured the bacterial decontamination capacity over time by comparing the standard system and the one capable of absorbing and releasing the mixture of antimicrobial active ingredients administered over time.

Test procedure: A scaffold (4 x 6 cm), prepared according to Example 1 and soaked with 1.3 mL of PSA, is immersed in a solution of RPMI 1640 medium w/L-Glutamine w/25 Mm Hepes (Biowest) and contaminated with a standardized antibiotic-sensitive strain of gram-positive Staph. Aureus bacterium in concentration 10⁶; the system thus prepared is placed in an incubator at 37 °C. After 24 hours, 72 hours and 7 days, the bacteria are transferred on a COS blood agar plate and after 3 days, bacterial growth is evaluated. The test was carried using the following conditions: a saline solution instead of PSA, a PSA in a solution of medium and bacteria, only the solution of medium and bacteria.

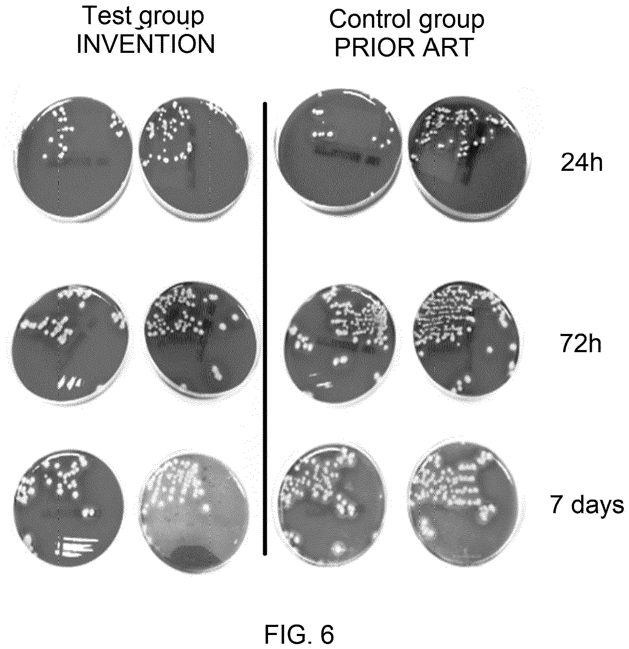

Fig. 6 shows the test results. The Test group uses a composite scaffold according to example 1, the Control group uses a commercial Pelnac-type scaffold. The dermal substitutes were soaked with a solution of antibiotics and then cultured with S. Aureus type bacteria. The number of CFUs that develops over time is lower in the Test group, thus demonstrating that the composite scaffold, releasing antibiotics over time, reduces the onset of infections.

As can be seen from Fig. 6 after 24 hours there is a significant reduction in the number of colony forming units (CFU) developed on the plate using the composite scaffold according to the invention in comparison with the reference. At 72 hours, the reduction is even more evident with an estimate of between 30-40% reduction. In Fig. 6 an evaluation at 7 days is also presented in which it is still noted how the inventive composite scaffold reduces the onset of bacterial colonies compared to the reference. The controls in which a physiological solution is used instead of PSA are not shown in Fig. 6, but the result was a massive and uniform bacterial proliferation in all cases.

The scaffold according to the invention can be used in reparative and reconstructive surgery which can involve various surgical specialties of depth, where the use of a porous and soft matrix is necessary. In this case the scaffold, in the form of a sheet, is fenestrated, that is, engraved with through slits. The slits may already be present in the scaffold or made by the surgeon with a scalpel, before implantation.

The scaffold according to the invention can be used in retro-pectoral and pre-pectoral breast reconstruction. If the scaffold is loaded with antibiotics and anti-inflammatories, the incidence of problems such as infections and capsular contracture can be reduced, thanks to the slow and prolonged release of antibiotics and anti-inflammatories from the scaffold after implantation.

The scaffold according to the invention is characterized by the ability to integrate into the soft tissues of the host, by means of normal biological processes and at the same time to prevent, by means of the slow release mechanism of antibiotics and anti-inflammatories, the development of serious infectious complications and chronic inflammation that can make repairing or reconstructive surgery useless. For these reasons, the scaffold according to the invention finds application in all the following surgical disciplines:
- breast surgery
- abdominal surgery
- urological surgery
- gynecological surgery
- neurosurgery
- peripheral nerve surgery
- orthopedics (joint surgery)
- cardio-vascular surgery
- Maxillofacial and oro-dental surgery
- plastic surgery and vulnology (dermal substitutes)

## Claims

1. Composite scaffold comprising:
- a bio-resorbable porous matrix and
- at least one type of lamellar solids with ion exchange capacity, incorporated in the bio-resorbable porous matrix or adhering to a surface of the bio-resorbable porous matrix; said lamellar solids being able to be loaded with active principles through ion exchange reactions,
**characterized in that**
said bio-resorbable porous matrix comprises collagen and/or microporous hyaluronic acid;
the percentage by weight of lamellar solids present in the composite scaffold is between 0.8-4.0%; and
the scaffold is sheet-shaped, suitable for implantation in a dermal lesion or soft tissue of a patient.

2. Scaffold according to claim 1, wherein the bio-resorbable porous matrix contains a mixture of lamellar solids comprising both lamellar solids having a cation exchange capacity (CEC) greater than 1 meq/g and lamellar solids having an anion exchange capacity (AEC) greater than 1 meq/g and said lamellar solids belong to one or more of the following chemical classes: hydrotalcites (double layered hydroxides or LDH), zirconium hydrogen phosphate of alpha, gamma or lambda type, double hydroxide salts (hydroxy double salts or HDS), phyllosilicates.

3. Scaffold according to any one of the preceding claims, wherein said composite scaffold has the shape of a sheet having a thickness of 0.1 - 20 mm.

4. Scaffold according to any one of the preceding claims, wherein said composite scaffold is loaded with one or more of the following active ingredients suitable for avoiding infections: antibiotics, antifungals, antibodies and antimicrobial agents including antiseptics and disinfectants.

5. Scaffold according to any one of the preceding claims, wherein said composite scaffold is loaded with one or more of the following active ingredients suitable for strengthening the patient's immune defenses against viruses and bacteria: antibodies such as immunoglobulins and vaccines.

6. Scaffold according to any one of the preceding claims, wherein said composite scaffold is loaded with one or more of the following active ingredients suitable for assisting skin and soft tissue regeneration: anti-inflammatories, growth factors, antibiotics and antifungals, natural extracts from placenta or colostrum.

7. Scaffold according to any one of the preceding claims, wherein said composite scaffold is loaded with active ingredients suitable for accelerating the cell regeneration process and improving the quality of the tissues, such as one or more of the following active ingredients: peptides and/or proteins such as glycoproteins, lipoproteins, cytokines.

8. Scaffold according to any one of the preceding claims, wherein said composite scaffold is loaded with active ingredients suitable for accelerating the cell regeneration process and improving the quality of the tissues, such as one or more of the following active ingredients: fragments of coding nucleotides including DNA, RNA, plasmids.

9. Scaffold according to any one of claims 5 to 8, further comprising a film of synthetic material removably applied to the sheet of the composite scaffold comprising the porous matrix and the lamellar solid.

10. Process for the production of a scaffold according to any one of the preceding claims comprising the following steps:
a. preparation of a suspension of one or more lamellar solids in a solvent;
b. application of the suspension to a bio-resorbable porous matrix; and
c. drying of the product obtained in step b;
wherein said step b of application of the suspension on the bio-resorbable porous matrix is carried out by means of a spray-coating process on one of the two sides of the bio-resorbable porous matrix.

11. Process according to claim 10, wherein said drying step c is carried out by freeze-drying or by evaporation of the solvent at reduced pressure, in the presence of desiccants or by evaporation in an oven or climatic chamber.

12. A process for producing a dermal substitute according to any one of claims 1 to 9, comprising the following steps:
a. preparation of a first phase consisting of one or more bio-resorbable polymers solubilized in a solvent;
b. preparation of a second phase consisting of a suspension of one or more lamellar solids in a solvent;
c. preparation of a third phase consisting of an organic solvent;
d. emulsion and homogenization of the three phases prepared in steps a, b and c;
e. drying of the product obtained in step d;
f. thermal and/or chemical crosslinking of the product obtained in step e and subsequent washing and drying.

13. Process according to claim 12, wherein in said steps a and b water is used as the solvent, and in said step c chloroform is used as organic solvent.

14. Process according to claim 12 or 13, wherein said drying step e is done by freeze-drying.

15. A process according to any one of claims 12 to 14, further comprising the step of adsorption of active ingredients in the composite scaffold by ion exchange reactions between the lamellar solids and a solution or suspension of active ingredients and in which said adsorption step of active ingredients in the composite scaffold occurs just prior to grafting the scaffold into the patient's dermal lesion or soft tissue.

## Patentansprüche

1. Verbundgerüst, umfassend:
- eine poröse, bioresorbierbare Matrix und
- mindestens eine Art von lamellaren Feststoffen mit ionenaustauschkapazität, die in die bioresorbierbare, poröse Matrix eingearbeitet sind oder an einer Oberfläche der bioresorbierbaren, porösen Matrix haften;
wobei die genannten lamellaren Feststoffe geeignet sind, mit Wirkstoffen durch lonenaustauschreaktionen beladen zu werden,
**dadurch gekennzeichnet, dass** die genannte poröse, bioresorbierbare Matrix Kollagen und/oder mikroporöse Hyaluronsäure umfasst;
der Gewichtsanteil der in dem Verbundgerüst enthaltenen lamellaren Feststoffe liegt zwischen 0,1-50,0%;
das Gerüst hat die Form eines Blattes, das geeignet ist, in eine dermale Läsion oder in ein Weichgewebe eines Patienten implantiert zu werden.

2. Gerüst nach Anspruch 1, wobei die bioresorbierbare poröse Matrix eine Mischung aus lamellaren Feststoffen enthält, die sowohl lamellare Feststoffe mit einer Kationenaustauschkapazität (CEC) von über 1 meq/g als auch lamellare Feststoffe mit einer Anionenaustauschkapazität (AEC) von über 1 meq/g umfasst, und wobei die lamellaren Feststoffe zu einer oder mehreren der folgenden chemischen Klassen gehören: Hydrotalcite (doppelschichtige Hydroxide oder LDH), Zirkoniumhydrogenphosphat vom Alpha-, Gamma- oder Lambda-Typ, Doppelhydroxidsalze (Hydroxydoppelsalze oder HDS), Phyllosilikate.

3. Gerüst nach einem der vorhergehenden Ansprüche, wobei das Verbundgerüst die Form eines Blattes mit einer Dicke von 0,1-20 mm hat.

4. Gerüst nach einem der vorhergehenden Ansprüche, wobei das Verbundgerüst mit einem oder mehreren der folgenden Wirkstoffe beladen ist, die zur Vermeidung von Infektionen geeignet sind: Antibiotika, Antimykotika, Antikörper und antimikrobielle Wirkstoffe einschließlich Antiseptika und Desinfektionsmittel.

5. Gerüst nach einem der vorhergehenden Ansprüche, wobei das Verbundgerüst mit einem oder mehreren der folgenden Wirkstoffe beladen ist, die zur Stärkung der Immunabwehr des Patienten gegen Viren und Bakterien geeignet sind: Antikörper wie Immunglobuline und Impfstoffe.

6. Gerüst nach einem der vorhergehenden Ansprüche, wobei das Verbundgerüst mit einem oder mehreren der folgenden Wirkstoffe beladen ist, die zur Unterstützung der Regeneration von Haut und Weichgewebe geeignet sind: Entzündungshemmer, Wachstumsfaktoren, Antibiotika und Antimykotika, natürliche Extrakte aus Plazenta oder Kolostrum.

7. Gerüst nach einem der vorhergehenden Ansprüche, wobei das Verbundgerüst mit Wirkstoffen beladen ist, die zur Beschleunigung des Zellregenerationsprozesses und zur Verbesserung der Qualität der Gewebe geeignet sind, wie beispielsweise einem oder mehreren der folgenden Wirkstoffe: Peptide und/oder Proteine wie Glykoproteine, Lipoproteine, Zytokine.

8. Gerüst nach einem der vorhergehenden Ansprüche, wobei das Verbundgerüst mit Wirkstoffen beladen ist, die zur Beschleunigung des Zellregenerationsprozesses und zur Verbesserung der Qualität der Gewebe geeignet sind, wie beispielsweise einem oder mehreren der folgenden Wirkstoffe: Fragmente codierender Nukleotide, einschließlich DNA, RNA, Plasmide.

9. Gerüst nach einem der Ansprüche 5 bis 8, das außerdem einen Film aus synthetischem Material umfasst, der entfernbar auf die Schicht des Verbundgerüsts aufgebracht ist, das die poröse Matrix und den lamellaren Feststoff umfasst.

10. Verfahren zur Herstellung eines Gerüsts nach einem der vorhergehenden Ansprüche, das die folgenden Schritte umfasst:
a. Herstellung einer Suspension aus einem oder mehreren lamellaren Feststoffen in einem Lösungsmittel;
b. Aufbringen der Suspension auf eine bioresorbierbare poröse Matrix; und
c. Trocknen des in Schritt b erhaltenen Produkts;
wobei Schritt b des Aufbringens der Suspension auf die bioresorbierbare poröse Matrix mittels eines Sprühbeschichtungsverfahrens auf einer der beiden Seiten der bioresorbierbaren porösen Matrix durchgeführt wird.

11. Verfahren nach Anspruch 10, wobei der Trocknungsschritt c durch Gefriertrocknung oder durch Verdampfen des Lösungsmittels bei reduziertem Druck, in Gegenwart von Trockenmitteln oder durch Verdampfen in einem Ofen oder einer Klimakammer durchgeführt wird.

12. Verfahren zur Herstellung eines Hautersatzes nach einem der Ansprüche 1 bis 9, umfassend die folgenden Schritte:
a. Herstellung einer ersten Phase, bestehend aus einem oder mehreren bioresorbierbaren Polymeren, die in einem Lösungsmittel gelöst sind;
b. Herstellung einer zweiten Phase, bestehend aus einer Suspension eines oder mehrerer lamellarer Feststoffe in einem Lösungsmittel;
c. Herstellung einer dritten Phase, bestehend aus einem organischen Lösungsmittel;
d. Emulsion und Homogenisierung der drei in den Schritten a, b und c hergestellten Phasen;
e. Trocknen des in Schritt
d erhaltenen Produkts;
f. thermische und/oder chemische Vernetzung des in Schritt d erhaltenen Produkts und anschließendes Waschen und Trocknen.

13. Verfahren nach Anspruch 12, wobei in den Schritten a und b Wasser als Lösungsmittel und in Schritt c Chloroform als organisches Lösungsmittel verwendet wird.

14. Verfahren nach Anspruch 12 oder 13, wobei der Trocknungsschritte durch Gefriertrocknung erfolgt.

15. Verfahren nach einem der Ansprüche 12 bis 14, das außerdem den Schritt der Adsorption von Wirkstoffen im Verbundgerüst durch lonenaustauschreaktionen zwischen den lamellaren Feststoffen und einer Lösung oder Suspension von Wirkstoffen umfasst, und bei dem der Schritt der Adsorption von Wirkstoffen im Verbundgerüst unmittelbar vor dem Einpflanzen des Gerüsts in die Hautläsion oder das Weichgewebe des Patienten erfolgt.

## Revendications

1. Échafaudage composite comprenant :
- une matrice poreuse biorésorbable et
- au moins un type de solides lamellaires dotés d'une capacité d'échange d'ions, incorporés dans la matrice poreuse bio-absorbable ou adhérant à une surface de la matrice poreuse bio-absorbable;
lesdits solides lamellaires étant capables d'être chargés d'ingrédients actifs par des réactions d'échange d'ions, **caractérisé en ce que** ladite matrice poreuse biorésorbable comprend du collagène et/ou de l'acide hyaluronique microporeux; le pourcentage en poids de solides lamellaires présents dans l'échafaudage composite est compris entre 0,1 et 50,0%; et l'échafaudage est en forme de feuille, approprié pour une implantation dans une lésion cutanée ou un tissu mou d'un patient.

2. Échafaudage selon la revendication 1, dans lequel la matrice poreuse biorésorbable contient un mélange de solides lamellaires qui comprend à la fois des solides lamellaires ayant une capacité d'échange de cations (CEC) supérieure à 1 meq/g et des solides lamellaires ayant une capacité d'échange d'anions (AEC) supérieure à 1meq/g et lesdits solides lamellaires appartiennent à une ou plusieurs des classes chimiques suivantes : hydrotalcites (layered double hydroxydes ou LDH), hydrogénophosphate de zirconium de type alpha gamma ou lambda, sels d'hydroxydes doubles (hydroxy double salts ou HDS), phyllosilicates.

3. Échafaudage selon l'une quelconque des revendications précédentes, dans lequel ledit échafaudage composite a la forme d'une feuille ayant une épaisseur de 0,1 à 20 mm.

4. Échafaudage selon l'une quelconque des revendications précédentes, dans lequel ledit échafaudage composite est chargé d'un ou plusieurs des ingrédients actifs suivants capables d'éviter les infections : antibiotiques, antifongiques, anticorps et agents antimicrobiens, parmi lesquels compris des antiseptiques et des désinfectants.

5. Échafaudage selon l'une quelconque des revendications précédentes, dans lequel ledit échafaudage composite est chargé d'un ou plusieurs des principes actifs suivants adaptés au renforcement des défenses immunitaires du patient contre les virus et les bactéries : des anticorps tels que des immunoglobulines et des vaccins.

6. Échafaudage selon l'une quelconque des revendications précédentes, dans lequel ledit échafaudage composite est chargé d'un ou plusieurs des ingrédients actifs suivants capables d'aider à la régénération de la peau et des tissus mous : anti-inflammatoires, facteurs de croissance, antibiotiques et antifongiques, extraits naturels du placenta ou colostrum.

7. Échafaudage selon l'une quelconque des revendications précédentes, dans lequel ledit échafaudage composite est chargé d'ingrédients actifs adaptés pour accélérer le processus de régénération cellulaire et améliorer la qualité des tissus, tels qu'un ou plusieurs des ingrédients actifs suivants : peptides et/ ou des protéines telles que les glycoprotéines, les lipoprotéines, les cytokines.

8. Échafaudage selon l'une quelconque des revendications précédentes, dans lequel ledit échafaudage composite est chargé d'ingrédients actifs capables d'accélérer le processus de régénération cellulaire et d'améliorer la qualité des tissus, tels qu'un ou plusieurs des ingrédients actifs suivants : fragments de codage nucléotides, notamment ADN, ARN, plasmides.

9. Échafaudage selon l'une quelconque des revendications 5 à 8, comprenant en outre un film de matière synthétique appliqué de manière amovible sur la feuille de l'échafaudage composite comprenant la matrice poreuse et le solide lamellaire.

10. Procédé de fabrication d'un échafaudage selon l'une quelconque des revendications précédentes comprenant les étapes suivantes : a. préparation d'une suspension d'un ou plusieurs solides lamellaires dans un solvant ; b. application de la suspension sur une matrice poreuse bio-résorbable ; et c. séchage du produit obtenu à l'étape b; dans lequel ladite étape b d'application de la suspension sur la matrice poreuse bio-résorbable est réalisée par un procédé de spray-coating sur l'une des deux faces de la matrice poreuse bio-résorbable.

11. Procédé selon la revendication 10, **caractérisé en ce que** ladite étape c de séchage est réalisée par lyophilisation ou par évaporation du solvant à pression réduite, en présence de dessicants ou par évaporation en étuve ou en enceinte climatique.

12. Procédé de production d'un substitut dermique selon l'une quelconque des revendications 1 à 9, comprenant les étapes suivantes: a. préparation d'une première phase constituée d'un ou plusieurs polymères bio-résorbables solubilisés dans un solvant; b. préparation d'une deuxième phase constituée d'une suspension d'un ou plusieurs solides lamellaires dans un solvant; c. préparation d'une troisième phase constituée d'un solvant organique; d. émulsification et homogénéisation des trois phases préparées aux étapes a, b et c ; e. séchage du produit obtenu à l'étape d ; f. réticulation thermique et/ou chimique du produit obtenu à l'étape e, lavage et séchage ultérieurs.

13. Procédé selon la revendication 12, dans lequel dans lesdites étapes a et b, de l'eau est utilisée comme solvant, et dans ladite étape c, du chloroforme est utilisé comme solvant organique.

14. Procédé selon la revendication 12 ou 13, dans lequel ladite étape de séchage est réalisée par lyophilisation.

15. Procédé selon l'une quelconque des revendications 12 à 14, comprenant en outre l'étape d'adsorption de principes actifs dans l'échafaudage composite au moyen de réactions d'échange d'ions entre les solides lamellaires et une solution ou suspension de principes actifs et dans lequel ladite étape de l'adsorption des ingrédients actifs dans la structure composite se produit immédiatement avant la greffe de la structure dans la lésion cutanée ou les tissus mous du patient.
